# FASCICULE DE BREVET EUROPEEN

(11) **EP 1 463 693 B1**
(45) Date de publication et mention de la délivrance du brevet: **09.11.2005**
(21) Numéro de dépôt: 02711925.4
(22) Date de dépôt: 08.01.2002
(51) Int. Cl.: C05F 17/02, G01N 33/00

(54) **DISPOSITIF OPTIMISE DE REGULATION ET DE MESURE DE LA TENEUR EN GAZ DANS LES PLATES-FORMES DE COMPOSTAGE OU DE TRAITEMENT DES DECHETS AVEC DES SONDES DE MESURE**
GASGEHALTSMESSVORRICHTUNG UND GASGEHALTSREGELVORRICHTUNG FÜR KOMPOST ODER FÜR ABFALLBEHANDLUNGSPLATTFORMEN MIT MESSPROBEN
OPTIMIZED DEVICE FOR THE REGULATION AND MEASUREMENT OF GAS CONTENT IN COMPOSTING PLATFORMS OR PLATFORMS FOR THE TREATMENT OF WASTE WITH MEASURING PROBES

(43) Date de publication de la demande: 06.10.2004
(73) Titulaire: Thurot, Philippe Jean Louis, 91250 Saintry (FR)
(72) Inventeur: Thurot, Philippe Jean Louis, 91250 Saintry (FR)
(86) Numéro de dépôt international: PCT/FR2002/000039
(87) Numéro de publication internationale: WO 2003/068709

(56) Documents cités:
- WO-A-97/19901
- DE-A- 2 940 739
- DE-A- 3 401 889
- GB-A- 2 037 432
- US-A- 3 138 448

## Description

La présente invention a pour objet un dispositif optimisé de régulation et de mesure de la teneur en oxygène ou tout autre gaz dans les plates-formes de compostage ou de traitement de déchets.

L'invention concerne plus particulièrement un dispositif de mesure discontinue dé la teneur en gaz, et notamment en oxygène, des andains de composts, déchets verts, déchets fermentescibles ou tout autre déchet dont on doit mesurer la teneur en gaz.

Dans le cadre de la présente invention, le terme « déchet » désigne indifféremment tous ces déchets.

Le dispositif de mesure est destiné à permettre de prendre la teneur en gaz au niveau d'une plate-forme de traitement de déchets, et en particulier des plates-formes de compostage ou des plates-formes de dépollution de sols pollués ; chaque andain formant un tas de déchets différencié ou un tas unique pouvant avoir un superficie importante de plusieurs centaines à plusieurs milliers dé mètres carrés.

Les déchets peuvent être notamment de nature ligneuses, fibreuses, boueuses ou terreuses, voire l'ensemble indifféremment.

Les plates-formes de traitement de déchets et notamment de compostage et de traitement des sols pollués, utilisent systématiquement une ou plusieurs sondes de mesure de la teneur en gaz et notamment de la teneur en oxygène ou CO2 avec le capteur directement intégré dans chaque sonde. La sonde comporte alors un capteur qui envoie le signal de mesure grâce à un transmetteur directement au système de programmation.

Les inconvénients de ces systèmes de mesure et régulation sont nombreux dès lors que les plates-formes sont grandes et qu'il convient de prendre la mesure des gaz et notamment de l'oxygène, au niveau de plusieurs andains ou à plusieurs endroits dans le tas de déchets.

Les capteurs sont des éléments sensibles, et il y a d'autant plus de risque que le matériel tombe en panne sur le site.

Il n'y a pas un seul capteur mais plusieurs capteurs de mesure permettant de réaliser la mesure en gaz dans le tas de déchets.

Les sondes de mesure sont coûteuses puisque chacune d'entre elles intègre un capteur, la mesure du signal et un dispositif de transmission du signal vers la régulation.

L'intégration de ces différents dispositifs dans la sonde oblige à la maîtrise des contraintes d'étanchéité sur site, pour protéger correctement la partie électronique des capteurs, de l'humidité et de la poussière.

Le signal transmis est de nature électrique, ce qui oblige à relier par un fil électrique, la sonde de mesure à la régulation et sa programmation.

Dans le cas des plates-formes de compostage ou de dépollution de sols, l'utilisation des engins retourneurs de déchets, favorise l'arrachement des fils électriques, sinon la casse des sondes de mesure et de leurs capteurs. WO 97/19901 décrit un system de compostage, dans laquel les conditions sont regulées. Le taux d'aération peut être régulé en corrélation avec la tempétature mesurée par les sondes thermiques placées aux endroits désirés dans le système.

L'utilisation des sondes et notamment des sondes à oxygène sur site, nécessite un calibrage régulier et systématique de toutes les sondes.

La ou les sondes doivent être alimentées en électricité pour fonctionner.

Ces inconvénients et ces problèmes conduisent à des frais élevés de maintenance et d'entretien, de calibrage des sondes, de non propreté quand il s'agit de déplacer les sondes, d'arrêt de fonctionnement des process lors de l'arrachement des fils électriques de liaison entre les sondes et la régulation.

Le but de l'invention est de proposer un dispositif de mesure et de régulation qui puisse palier ces inconvénients.

Le but de l'invention est atteint par un dispositif de mesure et de régulation des gaz et notamment de mesure de l'oxygène ou du CO2, fonctionnant en discontinu. Ce dispositif comprend un coffret de commande déporté de l'armoire centrale de régulation, comprenant un automate de régulation et programmation, des électro-vannes de régulation intégrées ou non à l'automate de programmation; une ou plusieurs cannes de prélèvement des gaz et notamment de la teneur en oxygène ou de celle en CO2; en particulier, pour la mesure du taux d'oxygène dans le tas de déchets, une sonde à oxygène du type à capteur à oxyde de Zirconium chauffé ; une pompe unique d'aspiration des gaz et notamment de l'oxygène ; une ou plusieurs cannes permettant de capter en profondeur les gaz à analyser présents dans l'air contenu dans le tas de déchets, et notamment la teneur en oxygène contenu dans le ou les tas de déchets.

Les capteurs à oxyde de zirconium réchauffés ont un temps de réponse très court, de l'ordre de quelques secondes (moins de dix secondes), et ne nécessitent, comme intervalle pour recalibrer la sonde, qu'un délai de l'ordre de six mois en fonctionnement normal.

Conformément à l'invention, le dispositif comprend aussi une ou plusieurs cannes de prélèvement des gaz et notamment de l'oxygène. Chaque canne comporte une crépine à l'intérieur de laquelle débouche un tuyau plastique qui traverse de part en part la canne.

Ce tuyau permet que l'air et le gaz dans le tas de déchets, soit aspiré à ce niveau. Le tube plastique de chaque canne, relie chaque canne aux électro-vannes de régulation intégrées dans l'armoire déportée. Les cannes sont éloignées de l'armoire déportée.

L'air contenu dans le tas de déchets est aspiré grâce à la pompe contenue dans l'armoire déportée. Le gaz produit par les déchets et contenu dans l'air est donc également aspiré jusqu'à l'armoire déportée où la teneur en gaz est analysée (sonde à Zirconium pour l'oxygène). La variation des paramètres liés au gaz, enclenche, en fonction des paramètres spécifiques liés au process, une régulation permettant de contrôler et maintenir les paramètres spécifiques d'exploitation de la plate-forme, à l'intérieur de valeurs données (exemple du maintien d'un taux d'oxygène entre deux valeurs cadres, pour un dispositif d'exploitation des plates-formes de compostage par aération pilotée utilisant des souffleries d'aération).

Le dispositif peut être utilisé, à la fois, pour mesurer la teneur d'un ou de plusieurs gaz contenus dans l'air et caractérisant ce tas ou tous les andains formant le tas ou les tas de déchets.

Les avantages de ce dispositif sont nombreux :
- Il n'y a plus aucun capteur de mesure de gaz sur les tas de déchets et dans les cannes (plus aucune sonde de mesure de gaz sur site, avec capteur, et notamment dans les zones de retournement des déchets).
- Une seule sonde de mesure de la teneur en gaz dans l'air pour un gaz donné, tel que l'oxygène ou le CO2, et totalement protégée dans l'armoire déportée, permettant de fournir les analyses de la teneur de ce gaz, pour toute une plate-forme, et successivement pour chaque andain dont on souhaite suivre les caractéristiques physico-chimiques.
- Les sondes sont supprimées. Désormais, on ne prélève le gaz qu'à partir de cannes « creuses » plongées dans le ou les tas de déchets.
- Il n'y a plus de liaison filaire et électrique entre la canne et la régulation, mais un simple 45 tuyau plastique servant au passage de l'air et du ou des gaz analysés, au niveau de l'armoire déportée.

Il peut être installé de nombreuses cannes, le coût de ce matériel étant très limité, par rapport à celui qui consisterait à installer dans chaque canne, un capteur de mesure. Et son transmetteur.

Avec un automate adapté intégrant des entrées et sorties du type PT 100 et PT 1000, il est désormais possible de supprimer également le transmetteur équipant habituellement les sondes à température, pour le pilotage des plates-formes de compostage et de dépollution.

Pour obtenir un bon fonctionnement du dispositif, un certain nombre de conditions doivent être avantageusement réunies :

Il faut impérativement une crépine au bout des cannes plongées dans les déchets sinon le tuyau plastique d'alimentation en air, risque d'être bouché ou colmaté.

Il faut une étanchéité parfaite depuis la crépine jusqu'aux électro-vannes intégrées à l'armoire de régulation ou directement couplées à l'automate de programmation; de même qu'il faut qu'aucun des tuyaux plastiques ne soient percés, sinon l'air qui sera aspiré au niveau de chaque canne, ne sera pas celui qui doit être analysé au coeur du tas de déchets ou de chaque tas de déchets, ou de chaque andain.

La pompe installée dans l'armoire déportée doit être d'une puissance suffisante pour aspirer rapidement l'air qui est successivement aspiré au niveau de chaque canne.

Les capteurs de la mesure en gaz dans l'air prélevé, doivent être suffisamment sensibles pour donner une mesure fiable et quasi immédiate de la teneur en gaz mesurée.

L'analyseur en gaz ne doit pas prendre en compte la teneur en gaz immédiatement aspirée et qui stagnait dans le tuyau plastique, au début du pompage, car cette donnée est fausse et ne correspond pas à la valeur qui doit être mesurée. Seule la valeur devant être mesurée, est celle obtenue et correspondant à l'air réellement aspiré à l'extérieur de la canne et n'ayant pas stagné dans le tuyau plastique.

La canne doit avoir une longueur suffisante permettant d'aller mesurer la teneur en gaz au coeur du tas de déchet.

L'automate de programmation pilotant l'ouverture et la fermeture des électrovannes permettant de pomper l'air et les gaz successivement prélevés dans chaque canne, doit être suffisamment performant pour commander les ouvertures et fermetures des vannes, les temps d'ouverture et fermetures de ces vannes, le temps pour une lecture du signal pertinent ; la lecture des signaux fournis par la sonde à gaz ou des sondes correspondant à d'autres gaz(exemple de sonde à oxygène ou de sonde à CO2) et les autres sondes de mesure (exemple des sondes à température).

Si l'on veut supprimer les transmetteurs concernant les sondes à température, l'automate de programmation dans l'armoire déportée doit intégrer des cartes analogiques d'entrée PT 100 ou PT 1000.

Pour protéger les matériels suivants (sonde ou sondes avec capteurs de mesure, la pompe d'aspiration, l'automate de régulation et programmation et les électrovannes de régulation), il est nécessaire de placer l'ensemble de ces matériels dans une ou plusieurs armoires déportées. Cette ou ces armoires déportées peuvent être séparées de l'armoire centrale qui pourra recueillir quant à elle, l'ensemble des données techniques fournies par le ou les capteurs de mesure.

Au niveau de la partie supérieure de la canne creuse, un presse étoupe ou un simple raccord suffit pour assurer le blocage et l'étanchéité parfaite entre la canne et le tuyau plastique d'aspiration de l'air et des gaz.

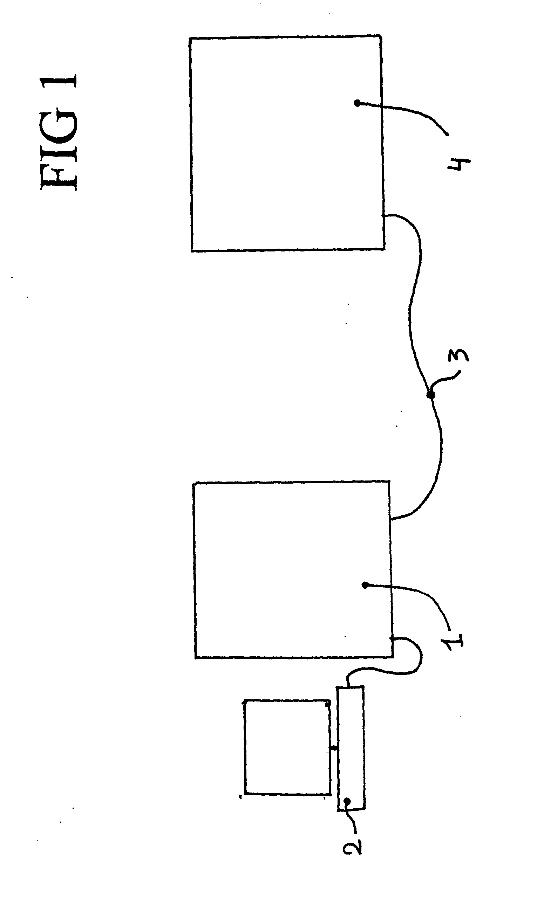

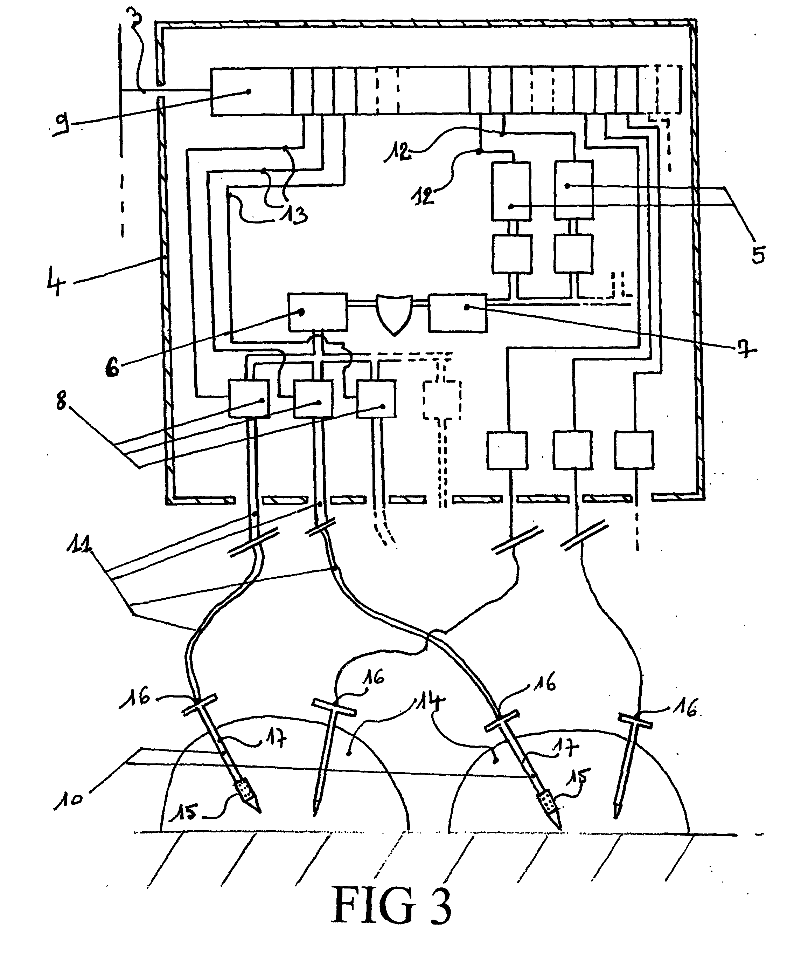

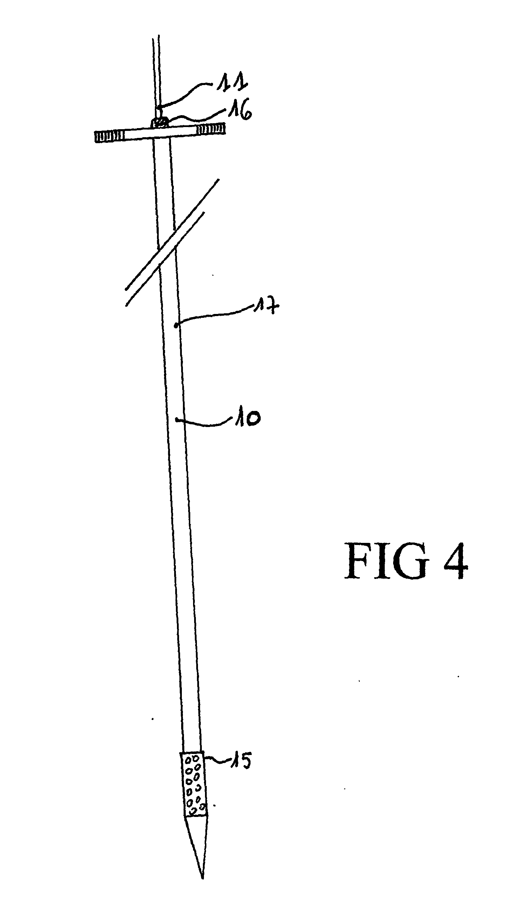

La description qui suit se reporte aux figures 1 à 4 qui représentent un dispositif de mesure de la teneur en gaz présent dans l'air présent à l'intérieur d'un tas de déchets, selon deux modes de réalisation préférés de l'invention.

L'installation comprend une armoire centrale de commande (1) comprenant l'unité centrale de régulation pouvant être couplée à un PC de commande ou ordinateur (2).
Elle comprend aussi, connectée par une liaison de type Bus (3), à cette armoire centrale (1), une armoire déportée (4) contenant une sinon plusieurs sondes de mesure (5) (une sonde par type de gaz analysé) ; elle comprend aussi une pompe d'aspiration du gaz (6) et un appareil permettant de réguler et régler le débit d'air alimentant la pompe (7) ; elle comprend plusieurs électro-vannes (8) pilotées par l'automate de programmation (9) (une électro-vanne par canne de prélèvement (10)). De préférence, la pompe d'aspiration du gaz (6) est équipée d'un appareil de régulation de débit d'air vers la ou les sonde(s), par exemple du type rotamètre.

La pompe d'aspiration (6) aspire l'air contenu successivement dans chacune des cannes (10) et passant obligatoirement par les tuyaux plastiques (11) reliant les cannes de prélèvement (10) de gaz aux électrovannes (8).

La pompe d'aspiration (6) est directement reliée à la sonde (5) telle que la sonde à oxygène, sinon aux autres sondes de mesure de gaz, par un tuyau d'alimentation (11).

La sonde ou les sondes à gaz (5) sont directement reliées par un câble électrique (12) à l'automate de programmation (9). Le câble transmet les mesures obtenue par chacune des sondes (5) à l'automate de programmation (9).

L'automate de programmation (9) est directement relié aux électrovannes par des câbles électriques (13) fournissant le signal électrique donnant l'ordre d'ouverture et fermeture des électrovannes (8).

Le prélèvement de gaz dans le ou les tas de déchets (14) s'effectue au moyen d'un canne traversée par un tuyau plastique (11).

Chaque tuyau plastique (11) relie une canne de prélèvement (10) à une électrovanne (8).

Une canne (10) comporte une crépine (15) à son extrémité et éventuellement un presse étoupe ou un raccord (16) à chaque extrémité du tube (17) formant la canne(10), et permettant ainsi de maintenir le tuyau plastique dans le corps de la canne.

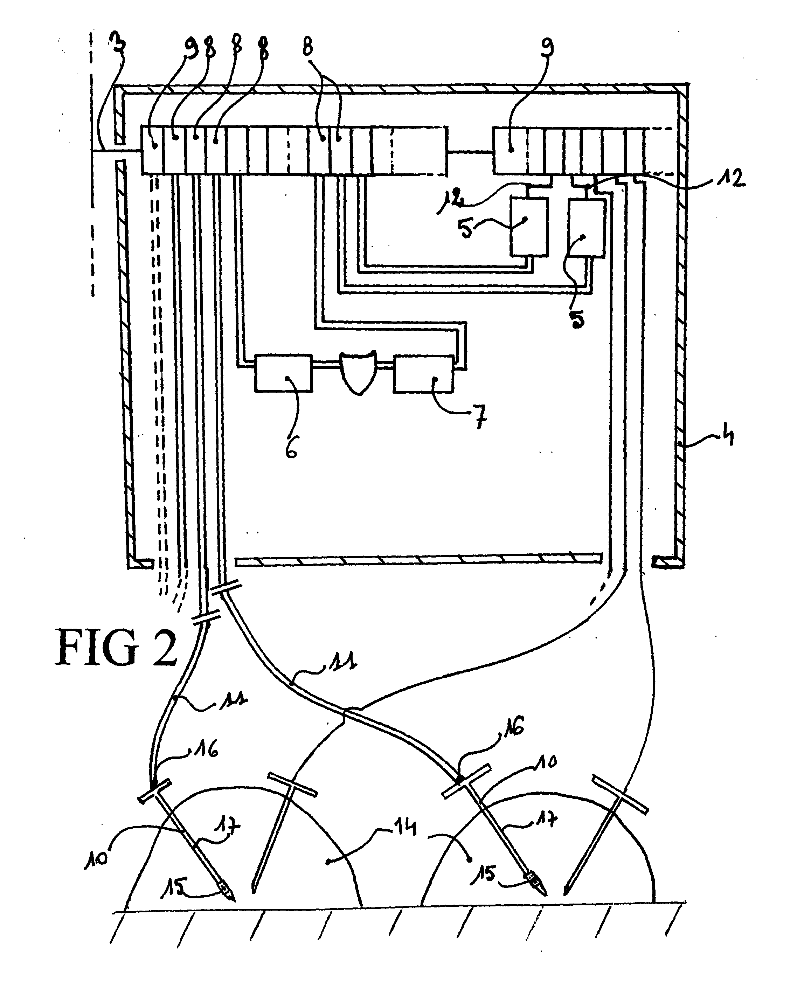

Selon un mode de réalisation représenté par la figure 2, l'unité de programmation peut comporter des cartes entrées et sorties intégrant directement les électrovannes et des cartes d'entrées et sorties du type PT 100 et PT 1000 (température) permettant de supprimer les transmetteurs équipant habituellement chaque sonde à température.

Il va sans dire que le nombre d'électro-vannes dépend de la taille des plates-formes et du nombre de mesures souhaité pour l'analyse.

Il va sans dire que l'armoire déportée peut contenir une seule sonde de mesure pour un seul type de gaz, comme elle peut contenir plusieurs sondes permettant d'analyser avec le même dispositif, plusieurs types de gaz, présents dans le ou les tas de déchets.

Selon une variante des modes de réalisation représentés, le matériel d'analyse intégré normalement dans une armoire déportée unique, peut être intégré dans une, deux ou plusieurs armoires déportées, en fonction des contraintes d'exploitation et de process.

Le présent dispositif a les nombreux avantages suivants :
- suppression des capteurs de mesure de gaz sur les tas de déchets et intégrés aux cannes ; simplicité, fiabilité et économie du dispositif ;
- facilité de remplacement des tuyaux plastiques en cas d'arrachement de ceux ci par les engins travaillant sur les plates-formes ;
- réduction du nombre de capteurs et notamment de capteurs servant à la mesure de la teneur en oxygène ou en CO2 pour le pilotage des plates-formes de compostage ou de traitement des déchets (un seul capteur sinon seulement deux ou trois capteurs peuvent suffire pour piloter des plates-formes de compostage de plusieurs dizaines de milliers de tonnes).
- possibilité d'avoir un seul capteur à oxygène pour piloter et assurer la régulation d'une plate-forme de compostage traitant plusieurs milliers de tonnes de déchets verts, boues et déchets fermentescibles, ou sols pollués.
- suppression possible du transmetteur du signal de température au niveau de chaque sonde de température.
- possibilité de posséder des cannes d'un seul modèle standard permettant de recevoir soit le tuyau plastique d'alimentation en air ou soit une sonde du type PT 100 ou PT 1000, ou tout autre type de capteur (capteur à humidité).
- la sonde d'analyse de l'oxygène dans le cas d'une plate-forme de compostage peut ne plus dériver et donc ne plus être aussi régulièrement comme par le passé, recalibrée (réétalonée).
- dans ce dispositif, si un réétalonage doit néanmoins être effectué, il ne concerne plus éventuellement qu'une seule sonde, voire deux ou trois sondes à oxygène seulement.

L'invention trouve toute une série d'applications privilégiées dans le domaine du compostage de tous les types de déchets organiques, et notamment des déchets verts, des boues et déchets fermentescibles, traités séparément ou mélangés entre eux.

Elle trouve aussi des applications privilégiées dans le domaine du traitement des sols pollués.

## Revendications

1. Dispositif optimisé de régulation et de mesure discontinue de la teneur en oxygène ou de tout autre gaz, dans les plates-formes de compostage ou de traitement des déchets, notamment sous forme d'andains, le dispositif comprenant au moins une armoire déportée (4) comprenant elle-même une ou plusieurs sondes de mesure du gaz (5) et, en particulier, au moins une sonde de mesure à oxygène ou CO₂ ; une pompe d'aspiration des gaz (6) ; des électrovannes (8) pilotées par un automate de programmation (9) ; une tuyauterie (11) qui relie chacune des électrovannes (8), à un élément de prélèvement de gaz, les électrovannes (8) couplées à la pompe (6) permettant successivement l'aspiration de l'air et des gaz contenus dans l'air au niveau de chaque élément de prélèvement (10) et l'envoi de cet air et des gaz à la ou les sondes de mesure (5), **caractérisé en ce que** l'élément de prélèvement est une canne (10) à deux extrémités opposées pouvant être plantée dans le ou les tas de déchets ou de compost ; chacune des cannes de prélèvement (10) correspondant à une seule et unique tuyauterie et comportant une crépine (15) d'aspiration de l'air à la première extrémité, la tuyauterie étant raccordée à la seconde extrémité de la canne et **en ce que** la sonde de mesure de l'oxygène (5) doit être capable de fournir dans un temps de réponse très court, la mesure de la teneur en oxygène de plusieurs andains et qu'il convient donc que cette sonde soit à capteur à oxyde de zirconium réchauffé, à temps de réponse très court, de l'ordre de quelques secondes et moins de dix secondes.

2. Dispositif selon la revendication 1, **caractérisé en ce que** les électrovannes (8) sont séparées de l'automate de programmation (9) ou sont intégrées directement à l'automate de programmation (9).

3. Dispositif selon l'une des revendications 1 ou 2, **caractérisé en ce que** le raccordement de la canne à la tuyauterie comporte un presse étoupe ou un raccord facilitant la fixation ou l'insertion de la tuyauterie.

4. Dispositif selon l'une des revendications 1 à 3, **caractérisé en ce qu'**une seule sonde de mesure du gaz (5), notamment l'oxygène ou le CO₂, peut permettre la mesure de la teneur, respectivement en oxygène ou CO₂, de plusieurs andains, grâce aux prélèvements obtenus à partir des différentes cannes de prélèvement de gaz (10).

5. Dispositif selon l'une des revendications 1 à 4, **caractérisé en ce que** l'automate de programmation (9) comprend des sondes de température et une ou plusieurs entrées-sorties informatiques lui permettant de recevoir des signaux du type PT 100 ou PT 1000 pour la mesure de température ou d'autres signaux pour la mesure des autres gaz présents.

6. Dispositif selon l'une des revendications 1 à 5, **caractérisé en ce que** la canne de prélèvement de l'air (10) dans les déchets, et servant à mesurer la teneur en oxygène, CO₂ ou de tout autre gaz, ne comporte pas de capteur, ni de transmetteur intégré au corps de la canne, mais seulement une crépine (15) et un tuyau d'alimentation en air (11).

7. Dispositif selon l'une des revendications 1 à 6, **caractérisé en ce que** la pompe d'aspiration du gaz (6) peut être équipée d'un appareil de régulation du débit d'air (7) vers la ou les sondes, du type rotamètre.

8. Dispositif selon l'une des revendications 1 à 7, **caractérisé en ce que** la came de prélèvement de l'air (10) comporte à l'une de ses extrémités une crépine de l'air (10) et à l'autre un presse-étoupe ou un raccord permettant l'insertion de la tuyauterie de prélèvement (11) de l'air dans le compost ou les déchets.

## Patentansprüche

1. Optimierte absatzweise Regelungs- und Messvorrichtung für den Sauerstoffgehalt oder einen beliebigen Gasgehalt auf den Kompostier- oder Abfallbehandlungsstellen insbesondere als Schwaden, wobei die Vorrichtung mindestens einen verlagerten Schrank (4) umfasst der seinerseits eine oder mehrere Gasmesssonden (5) und insbesondere mindestens eine Sauerstoff- oder CO2-Messsonde; eine Gasansaugpumpe (6); von einem Programmierautomaten (9) gesteuerte Magnetventile (8) ; eine Rohrleitung (11) die jedes der Magnetventile (8) mit einem Element für Gasprobeentnahmen verbindet umfasst, wobei die mit der Pumpe gekoppelten Magnetventile (8) auf Höhe eines jeden Probeentnahmeelements (10) aufeinanderfolgend eine Ansaugung von Luft oder von den in der Luft enthaltenen Gase, sowie die Zuführung dieser Luft oder dieser Gase zur Meassonde oder zur Messsonden (5) ermöglicht, **dadurch gekennzeichnet dass** das Probeentnahmeelement ein Rohrstock (10) mit zwei gegenüberliegenden Endstücken ist, die in den Abfall- oder in den Komposthaufen eingeführt werden können; wobei jeder Probeentnahmerohrstock (10) einer einzigen und alleinigen Rohrleitung entspricht und am ersten Endstück einen Luftsaugkopf (15) aufweist, wobei die Rohrleitung mit dem zweiten Endstück des Rohrstocks verbunden ist und dass die Sauerstoffmesssonde (5) in der Lage sein muss innerhalb einer sehr kurzen Ansprechzeit den Messwert für den Sauerstoffgehalt in mehreren Schwaden zu liefern und dass es dementsprechend erforderlich ist, dass dies eine Sonde mit erwärmten Zirkonoxidsensor, mit sehr kurzer Ansprechzeit in der Größenordnung von einigen Sekunden und weniger als zehn Sekunden sein muss.

2. Vorrichtung nach Anspruch 1, **dadurch gekennzeichnet dass** die Elektroventile (8) vom Programmierautomaten (9) getrennt, oder unmittelbar im Programmierautomaten (9) eingebaut sind.

3. Vorrichtung nach Anspruch 1 oder 2, **dadurch gekennzeichnet, dass** der Anschluss zwischen dem Rohrstock und der Rohrleitung eine Stopfbüchse oder ein Anschlusstück aufweist, die die Befestigung oder die Einführung der Rohrleitung erleichtern.

4. Vorrichtung nach einem der Ansprüche 1 bis 3, **dadurch gekennzeichnet dass** eine einzige Gasmesssonde (5), insbesondere für Sauerstoff oder CO₂ die Gehaltsmessung, jeweils von Sauerstoff oder von CO₂ in mehreren Schwaden mit Hilfe der mit den verschiedenen Gasprobeentnahmerohrstöcken (10) erhaltenen Proben ermöglichen kann.

5. Vorrichtung nach einem der Ansprüche 1 bis 4, **dadurch gekennzeichnet dass** der Programmierautomat (9) Temperaturfühler sowie einen oder mehrere elektronischen Eingänge/Ausgänge umfasst, die es ermöglichen Signale der Art PT 100 oder PT 1000 für die Temperaturmessung oder sonstige Signale zur Messung der sonstigen vorhandenen Gase zu empfangen.

6. Vorrichtung nach einem der Ansprüche 1 bis 5, **dadurch gekennzeichnet dass** der Luftprobeentnahmerohrstock (10) in den Abfällen, der dazu dient den Sauerstoff-, den CO₂-Gehalt oder einen beliebigen Gasgehalt zu messen keinen im Rohrstockkörper eingebauten Fühler oder Messwertgeber, sondern nur einen Saugkopf (15) und eine Luftzufuhrleitung (11) aufweist.

7. Vorrichtung nach einem der Ansprüche 1 bis 6, **dadurch gekennzeichnet, dass** die Gasansaugpumpe (6) mit einem Luftdurchflussreguliergerät (7) zu der oder zu den Sonden, der Art eines Rota-Durchflussmessers ausgerüstet werden sein kann.

8. Vorrichtung nach einem der Ansprüche 1 bis 7, **dadurch gekennzeichnet, dass** der Luftprobeentnahmerohrstock (10) an einem seiner Endstücke einen Luftsaugkopf (10) und am anderen eine Stopfbüchse oder ein Anschlussstück aufweist, die die Einführung der Luftentnahmerohrleitung (11) in den Kompost oder in die Abfälle ermöglichen.

## Claims

1. Optimized device for controlling and intermittently measuring the content of oxygen, or any other gas, on composting or waste treatment platforms, notably using the windrow system. The device comprises at least one independent cabinet (4) which, in turn, comprises one or more gas measuring probes (5) and, in particular, at least one oxygen or CO2 measuring probe; a gas suction pump (6); solenoid valves (8) driven by an automatic programming control system (9); a pipe (11) which connects each of the solenoid valves (8) to a gas sampling unit, the solenoid valves (8) coupled to the pump (6) are used to successively suck in the air and gases contained in the air surrounding each sampling unit (10) and dispatch said air and gases to the measuring probe(s) (5). Said device is **characterized in that** the sampling unit is a double-ended rod (10) which can be driven into the pile(s) of waste or compost; each of the sampling rods (10) corresponds to a single, solitary pipe and comprises an air intake filter (15) on one end of the rod, the pipe being connected to the other end. It is also **characterized in that** the oxygen measuring probe (5) shall be capable of supplying the oxygen content measurement for several windrows in a very short response time, and that said probe should, therefore, have a heated zirconium oxide sensor with a very short response time, in the region of a few seconds and less than ten seconds.

2. Device according to claim 1, **characterized in that** the solenoid valves (8) are separated from the automatic programming control system (9) or are directly integrated in the automatic programming control system (9).

3. Device according to claim 1 or 2, **characterized in that** the connection of the rod to the pipe comprises a gland or a connector making it easy to attach or insert the pipe.

4. Device according to any one of claims 1 to 3, **characterized in that** a single gas measuring probe (5), notably oxygen or CO2, can be used to respectively measure the content of oxygen or CO2 of several windrows, thanks to the samples obtained from the different gas sampling rods (10).

5. Device according to any one of claims 1 to 4, **characterized in that** the automatic programming control system (9) comprises temperature probes and one or more computer input/output port which it can use for receiving PT 100 or PT 1000 signals for measuring the temperature or other signals for measuring the other gases present.

6. Device according to any one of claims 1 to 5, **characterized in that** the rod (10) for sampling the air in the waste, and for measuring the content of oxygen, CO2 or any other gas, does not comprise any sensors, or transmitters integrated into the body of the rod, but only a filter (15) and an air inlet pipe (11).

7. Device according to any one of claims 1 to 6, **characterized in that** the gas suction pump (6) can be fitted with a rotameter (7) for monitoring the air flow rate to the probe(s).

8. Device according to any one of claims 1 to 7, **characterized in that** one of the ends of the air sampling rod (10) comprises an air filter (10), and the other a gland or a connector making it possible to insert the air sampling pipe (11) into the compost or waste.
